# EUROPEAN PATENT SPECIFICATION

(11) **EP 4 251 242 B1**
(45) Date of publication and mention of the grant of the patent: **15.04.2026**
(21) Application number: 21752151.7
(22) Date of filing: 30.06.2021
(51) Int. Cl.: A61M 5/315, A61M 5/28

(54) **KIT FOR THE SUCCESSIVE AND COAXIAL INJECTION OF A MEDICAMENT**
KIT ZUR AUFEINANDERFOLGENDEN UND KOAXIALEN INJEKTION EINES MEDIKAMENTS
KIT POUR L'INJECTION SUCCESSIVE ET COAXIALE D'UN MÉDICAMENT

(30) Priority: 27.11.2020 IT 202000028709
(43) Date of publication of application: 04.10.2023
(73) Proprietor: GEM S.r.l., 55049 Viareggio (LU) (IT)
(72) Inventor: FACCIOLI, Giovanni, 37066 Sommacampagna (Verona) (IT); SOFFIATTI, Renzo, 37066 Sommacampagna (Verona) (IT); TESSARI, Lorenzo, 37019 Peschiera del Garda (Verona) (IT); TESSARI, Mirko, 37019 Peschiera del Garda (Verona) (IT)
(74) Representative: Dondi, Silvia
(86) International application number: PCT/IB2021/055855
(87) International publication number: WO 2022/112858

(56) References cited:
- EP-A1- 1 093 826
- EP-A1- 1 685 865
- EP-A2- 2 168 619
- EP-B1- 1 093 826
- US-A1- 2003 120 201
- US-A1- 2017 361 019

## Description

### TECHNICAL FIELD OF THE INVENTION

The present invention relates to a kit for the subsequent injection and/or subsequent insertion of substances and/or elements in the human body in a coaxial manner.

More particularly, the present invention relates to a kit for the subsequent injection and/or subsequent insertion of substances and/or elements into the human body, such as for example the same substance or two different substances at successive times in the same injection site or a substance and a different element, such as a further device, in successive times at the same injection site.

### PRIOR ART

In the medical field, it is very often useful to insert, for example inject, a same substance or more substances into the same implantation site at successive times.

In order to do this, for example there arises the need to inject a first substance into a given site and then to be able to inject the second substance (or in any case to carry out the second injection) exactly into the same site.

Clearly, this is a very difficult operation, which requires great precision for its success, especially when the area to be injected is small in size.

An example of such a method is that relating to the treatment of varices or varicose veins, wherein a method for the reduction thereof provides for the injection - into the vein to be treated - of a surfactant substance (or surfactant sclerosant such as for example sodium tetradecyl sulphate known by the trade name Fibro-Vein^{®} or Trombovar^{®} or the oxy-polyethoxy dodecane known by the trade name Atossisclerol^{®} or Sotradecol^{®}) which causes the vein to contract and subsequent injection - using the same needle and therefore exactly at the same site of the human body - of an adhesive substance which allows to seal the bloodstream by gluing the inner walls of the venous tract of interest.

Such technique is also known as sclerotherapy with "mousse" or as "scleromousse" (tourbillon technique). This technique is officially identified by the ruling issued by AIFA (Italian Medicines Agency) on Fibro-Vein^{®} in 2015 and on Atossisclerol^{®} in 2019.

The surfactant sclerosant allows to increase, with the same drug concentration, the contact of the sclerosant with the endothelium of the vein.

In such technique, two disposable plastic syringes and a special connector, still made of disposable plastic, are used to mix (by passing from one syringe to another by means of the connector), the biocompatible air or gases and the surfactant sclerosant, at the selected doses. A dense and compact mousse, which can be injected at the prescribed doses, comprising microbubbles of gas and drug, is obtained in a few seconds and through about ten steps. The size of such microbubbles may depend on several factors, such as the type of connector, the syringes used, etc...., which are linked to the specific requirements and techniques of manufacture.

It is a procedure that requires great dexterity and attention by the surgeon so as to ensure the success of the operation.

Such procedure is also required in other medical fields, as mentioned, for inserting two different substances one after the other or the same substance at two different times into the human body.

There is therefore a need for a device or kit that allows to carry out the actions indicated above - and also others that generally meet the same principle - in an easier and safer manner.

### OBJECTS OF THE INVENTION

The object of the present invention is to improve the state of the art in the field of devices for the injection and/or insertion of substances and/or elements into the human body.

In the scope of such technical task, an object of the present invention is to provide a kit for the subsequent injection and/or subsequent insertion of substances and/or elements which is practical to use and cost-effective.

A further object of the present invention is to provide a kit for the subsequent injection and/or the subsequent insertion of substances and/or elements that can guarantee exactly the same injection/insertion site while having to carry out these operations in successive times with respect to each other.

Still another object of the present invention is to provide a kit for the subsequent injection and/or subsequent insertion of substances and/or elements that can be used by medical personnel simply and quickly.

According to the present invention, provided herein is a kit according to claim 1.

The dependent claims refer to preferred and advantageous embodiments of the invention.

### BRIEF DESCRIPTION OF THE DRAWINGS

Further characteristics and advantages of the present invention will be more apparent from the detailed but non-exclusive description of a preferred embodiment of a kit for subsequent injection and/or subsequent insertion of substances and/or elements into the human body according to the present invention, provided by way of non-limiting example in the attached drawings, wherein:

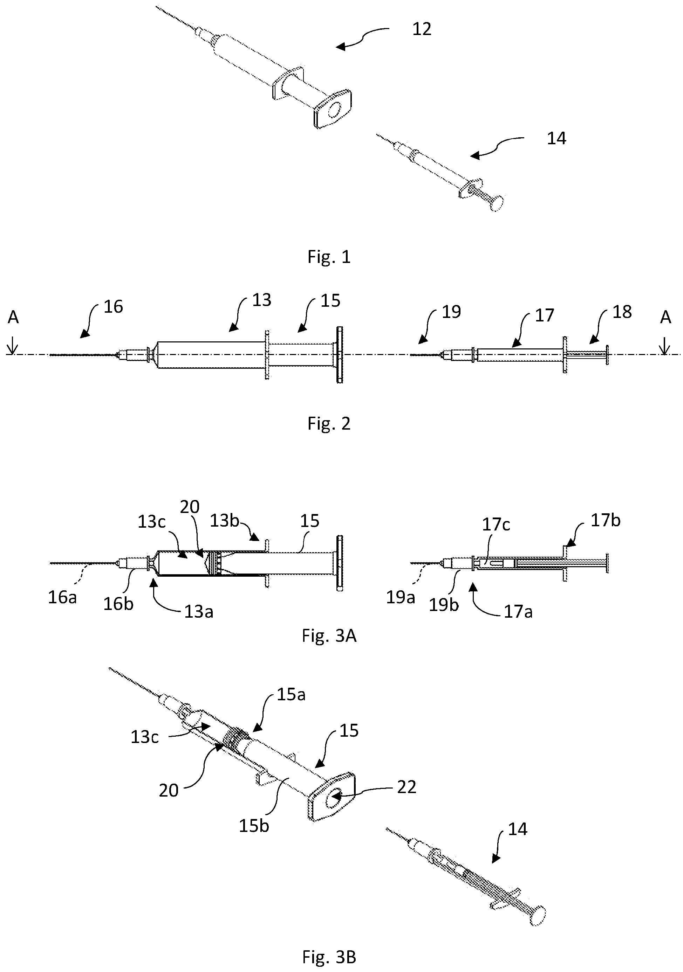

figure 1 is a rear perspective view of a kit according to the present invention in a version in which the two components of the kit are separated from each other;
figure 2 is a lateral view of the kit of figure 1;
figure 3A is an only partially sectional lateral view of the kit of figure 2, taken along the plane of line A-A;
figure 3B is a rear perspective and only partially sectional view of the kit of figure 2, taken along the plane of line A-A;
figure 4 is a lateral view of the kit according to the preceding figures according to an operative step;
figure 5A is an only partially sectional lateral view of the kit of figure 4 taken along the plane of line A-A;
figure 5B is a rear perspective and only partially sectional view of the kit of figure 4, taken along the plane of line B-B;
figure 6 is a rear perspective view of the kit according to the present invention wherein the two components of the kit are in the assembled version;
figure 7 is a lateral view of the kit of figure 6;
figure 8A is an only partially sectional lateral view of the kit of figure 7, taken along the plane of line C-C;
figure 8B is a rear perspective and only partially sectional view of the kit of figure 7, taken along the plane of line C-C;
figure 9 is a view of an enlarged detail and a sectional view of the kit of figure 8A;
figure 10A is a partially sectional lateral view of a version of the kit for the subsequent injection and/or subsequent insertion of substances and/or elements according to the present invention;
figure 10B is a sectional lateral view of the kit of figure 10A, taken along the plane of line D-D;
figure 10C is a view of an enlarged detail of the kit of figure 10A;
figures 11A and 11B are two perspective views of the detail of figure 10C, respectively in assembled and disassembled version with a further element of the kit of figure 10A;
figure 12A is a partially sectional front perspective view of a further version of the kit for the subsequent injection and/or subsequent insertion of substances and/or elements according to the present invention;
figure 12B is a lateral view from the side of the kit of figure 12A;
figure 12C is a top lateral view of the kit of figure 12A;
figure 12D is a perspective view of an enlarged detail of the kit of figure 12A;
figures 12E and 12F are respectively lateral and perspective views of an element of the kit of figure 12A;
figure 13 is an exploded view of the kit according to the present invention;
figure 14 is an exemplifying lateral view of a kit for the subsequent insertion of at least one substance and at least one element according to the present invention, in which the second component of the kit is represented in two different operative versions;
figure 15 is a lateral view of a further version according to the present invention of a kit for the subsequent injection of substances in which the two components of the kit are combined together,
figure 16 is a lateral view of a further version of a kit according to the present invention.

### EMBODIMENTS OF THE INVENTION

With reference to the attached figures, a kit for the subsequent injection and/or the subsequent insertion of substances and/or elements into the human body is indicated in its entirety with reference number 10.

According to the present treatise, the expression "injection" and the expression "insertion", as well as the expression "substance" and the expression "element" will be used as synonyms, unless specified otherwise.

Furthermore, unless otherwise specified the expression "fluid" is used to indicate a liquid substance, gaseous substance, plasma, etcetera, or a combination thereof.

The kit 10, in a first version thereof, comprises a first syringe element 12 and at least one second syringe element 14.

The expression "subsequent injection" or "subsequent insertion" into a site of the human body is used to indicate that the first syringe element 12 is adapted in use to determine the site of injection and/or insertion into the human body and the second syringe element 14 is suitable in use to inject or insert at least one substance or at least one element into the same injection/insertion site determined by the first syringe element. Therefore, the expression "subsequent" means that it comes after the injection and/or the insertion or suctioning carried out by the first syringe element 12, as will be better explained hereinafter.

According to the version illustrated in the figures exclusively by way of example, the first syringe element 12 comprises a 5 ml syringe while the second syringe element 14 comprises a 2 ml syringe.

Naturally, different capacities for the first 12 and second 14 syringe element may also envisaged. However, the first syringe element 12 has a greater capacity than the second syringe element 14.

Furthermore, the first syringe element 12 is capable of housing the second syringe element 14 at least partially therein.

In at least one embodiment of the present invention, the kit 10 exclusively consists of the first syringe element 12 and of the second syringe element 14, obviously besides the substances, for example medical substances, and/or elements which can be contained therein and which can be inserted into the human body.

As a matter of fact, the first syringe element 12 and the second syringe element 14 are suitable to contain at least one substance and/or at least one element to be inserted into the human body, such as for example a substance in the fluid or liquid state to be injected into the human body, an element 30 comprising an optical fibre device, a laser fibre device, a Radio Frequency (RF) device or antenna, a device capable of transporting and/or dispensing steam or other suitable gas, for example being tubular-shaped, a device capable of generating heat and/or radiation, for example in the infrared spectrum, or microwaves, etcetera.

In a version of the invention, the substance contained in the first syringe element 12 is for example a medical substance such as for example a biocompatible surfactant substance (such as, for example, a surfactant sclerosant such as sodium tetradecyl sulphate known by the trade name Fibro-Vein^{®} or Trombovar^{®} or oxy-polyethoxy dodecane known by the trade name Atossisclerol^{®} or Sotradecol^{®} etcetera) while the substance contained in the second syringe element 14 is for example a medical substance such as a biocompatible adhesive agent, for example based on cyanoacrylate, such as for example the one known by the trade name Loctite-Attak.

The maintenance of the injection/insertion position is extremely important also due to the fact that, for example as regards Loctite-Attak but also for other medical substances or adhesive agents, it acts by gluing only if the surfaces to be joined are in contact with each other, and therefore only after the inner walls of the veins to be treated (should such kit be used as an alternative for the sclerotherapy procedure indicated above) were collapsed by means of the first medical substance or by means of the surfactant agent.

In a further version, the substance contained in the first syringe element 12 is the same as that contained in the second syringe element 14, possibly having different dose and/or different density or the same density.

In a further version of the invention, the substances present in the first 12 and/or second 14 syringe element may be biocompatible cementing elements.

In a further version of the invention, the first syringe element 12 may be used to suction a biological liquid present at a given surgical site while the second syringe element 14 can be used to inject, exactly at the suctioning site, the substance contained therein.

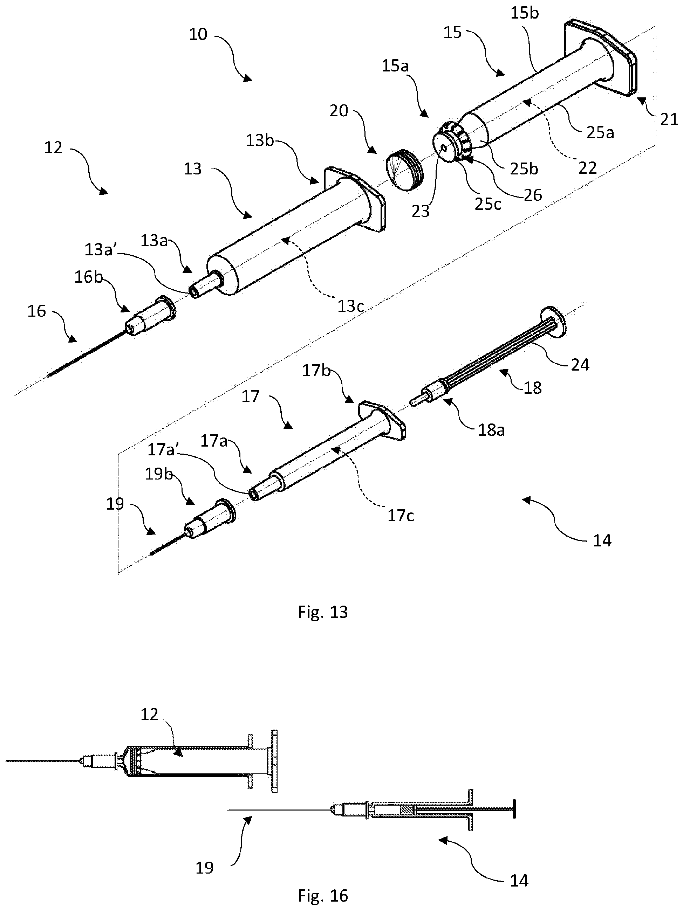

In this case, the second syringe element 14 can be provided with a second extra-long needle 19 capable of piercing (as better described hereinafter and as visible in figure 16) the plunger 15 and/or the capsule or gasket 20 of the head 15a of the plunger 15 and overcome the possible liquid suctioned by the first syringe element 12. Therefore, a bicomponent resin such as bone cement, possibly based on liquid poly(methyl methacrylate) (PMMA) can be inserted into the second syringe element 14 and injected into the vein or into the injection site where, after a few minutes, hardening, it blocks any blood outflow.

Getting into the detail of an embodiment of the present invention, the first syringe element 12 comprises an internally hollow cylindrical body 13, a plunger 15 (or first piston 15) and a first needle 16. The plunger 15 slides inside the cylindrical body 13, along a longitudinal direction determined precisely by the cylindrical body 13 and/or by a longitudinal axis of the first syringe element 12.

As a matter of fact, the first syringe element 12 extends along a longitudinal axis (first longitudinal axis) which can also be the symmetry axis thereof.

The second syringe element 14 extends along a longitudinal axis (second longitudinal axis) which can also be the symmetry axis thereof and which - in use - coincides with the longitudinal axis of the first syringe element 12.

Similarly, the second syringe element 14 comprises an internally hollow cylindrical side wall 17, a piston 18 (or second piston 18) and a second needle 19.

The first needle 16 and the second needle 19 are internally hollow and/or comprise respectively a first inner lumen 16a and a second inner lumen 19a.

There is also present a first connection element 16b between the first needle 16 and a first end 13a of the cylindrical body 13 to connect, possibly removably, the first needle 16 and the cylindrical body 13 of the first syringe element 12.

Similarly, there may be present a second connection element 19b between the second needle 19 and a tip 17a of the cylindrical side wall 17 to connect, possibly removably, the second needle 19 and the cylindrical side wall 17 of the second syringe element 14. The first connection element 16b and/or the second connection element 19b may comprise a Luer lock.

The cylindrical body 13 determines a first end 13a, to which the first needle 16 is constrained, possibly removably, and a second end 13b, opposite the first end 13a. The second end 13b is the open end of the cylindrical body 13, from which the cavity 13c inside the cylindrical body 13 can be accessed in use.

On the other hand, the first end 13a is an end closed by the first needle 16 but placed in fluid communication with the cavity 13c and with the inner lumen 16a of the first needle 16.

The first end 13a has, in at least one version of the invention, a tubular shape, for example cylindrical or truncated cone, having a cross-section, for example circular, and it is fitted into a special seat for housing the first connection element 16b.

The plunger 15 hermetically slides inside the cylindrical body 13 and/or inside the cavity 13c thereof.

The cylindrical side wall 17 comprises a tip 17a, to which there is constrained, possibly removably, the second needle 19, and a distal end 17b, opposite to the tip 17a. The distal end 17b is the open end of the cylindrical side wall 17, from which the space 17c inside the cylindrical side wall 17 can be accessed in use.

On the other hand, the tip 17a is an end closed by the second needle 19 but placed in fluid communication with the space 17c and with the inner lumen 19a of the second needle 19.

The tip 17a has, in at least one version of the invention, a tubular shape, for example cylindrical or truncated cone, for example having a circular cross-section, and it is fitted into a special seat for housing the second connection element 19b.

Therefore, both the first end 13a and the tip 17a have - therein - a connection channel 13a', 17a' respectively with the first lumen 16a of the first needle 16 and with the second lumen 19a of the second needle 19, as well as with the cavity 13c and with the space 17c.

The piston 18 slides, for example hermetically, inside the space 17c.

Contained in the cavity 13c and/or the space 17c is at least one substance to be injected into the human body and/or the at least one element 30 to be inserted into the human body (as will be described in greater detail hereinafter).

The at least one substance and/or the at least one element flows through the inner lumen 16a of the first needle 16 and/or through the inner lumen 19a of the second needle 19, pushed respectively by the plunger 15 and/or by the piston 18.

While the piston 18 is generally, in at least one version of the invention, of the known type, the plunger 15 is peculiar according to the present invention.

**In** particular, the plunger 15 has a head 15a, which is suitable in use, when fully inserted into the cavity 13c and/or into the cylindrical body 13, to internally come into contact with the first end 13a of the cylindrical body 13.

The head 15a is faced toward the first needle 16.

**In** addition, the plunger 15 has a stem 15b. The stem 15b is internally hollow and/or it comprises an internal channel 22. The channel 22 is a channel passing from the head 15a of the plunger 15 up to an end part 21 thereof.

The plunger 15 and/or the head 15a of the plunger 15 comprises a capsule or gasket 20. The capsule or gasket 20 is generally made of rubber and/or it has a substantially conical shape or in any case corresponding to the inner area of the cylindrical body 13 at the first end 13a thereof.

The capsule or gasket 20 is hermetically sealed in the sense that it allows the sliding of the plunger 15 inside the cavity 13c of the cylindrical body 13 but, at the same time, it prevents the solution contained in the cavity 13c from flowing out from the side of the capsule or gasket 20.

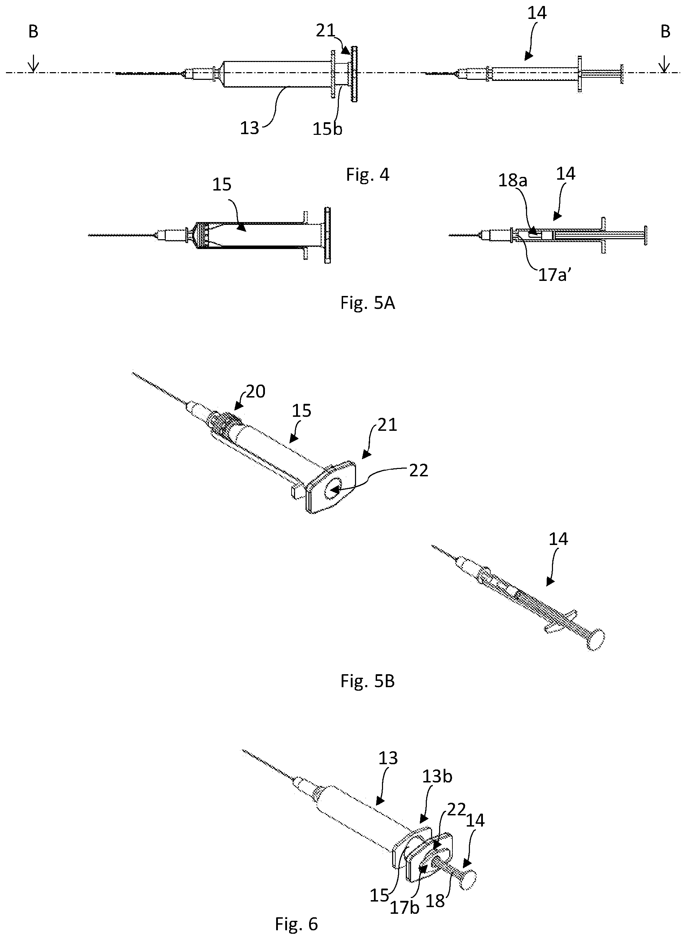

When the plunger 15 is fully inserted into the cavity 13c of the cylindrical body 13, the head 15a and/or the capsule or gasket 20 is internally in contact with the first end 13a of the cylindrical body 13 and the solution inserted into the cavity 13c is made to fully flow out of the first needle 16. Such conformation is for example visible in figures 4, 5A and 5B.

**In** this case, the stem 15b is almost fully housed inside the cavity 13c, except for the end part 21 part. As observable from such figures, the end part 21 is shaped so as to allow it to be gripped and/or moved, for example by two operator fingers, and for example it is flanged.

As mentioned, the stem 15b of the plunger 15 has a hollow internal channel 22.

Such internal channel 22 is arranged coaxially with the stem 15b and/or it is positioned parallel and/or coaxial with respect to the longitudinal direction of the first syringe element 12 and/or to the sliding direction of the plunger 15.

Such internal channel 22 is shaped so as to allow the housing of the second syringe element 14 therein and/or in the recess determined by it. The conformation of the internal channel 22 is therefore suitable to allow the insertion of the second syringe element 14 thereinto and the subsequent exit.

In particular, in at least one version of the invention, the internal channel 22 has a conformation corresponding to and/or complementary to that of the second syringe element 14, but slightly larger in size specifically so as to allow the movement of the latter and/or the housing thereof, possibly removable.

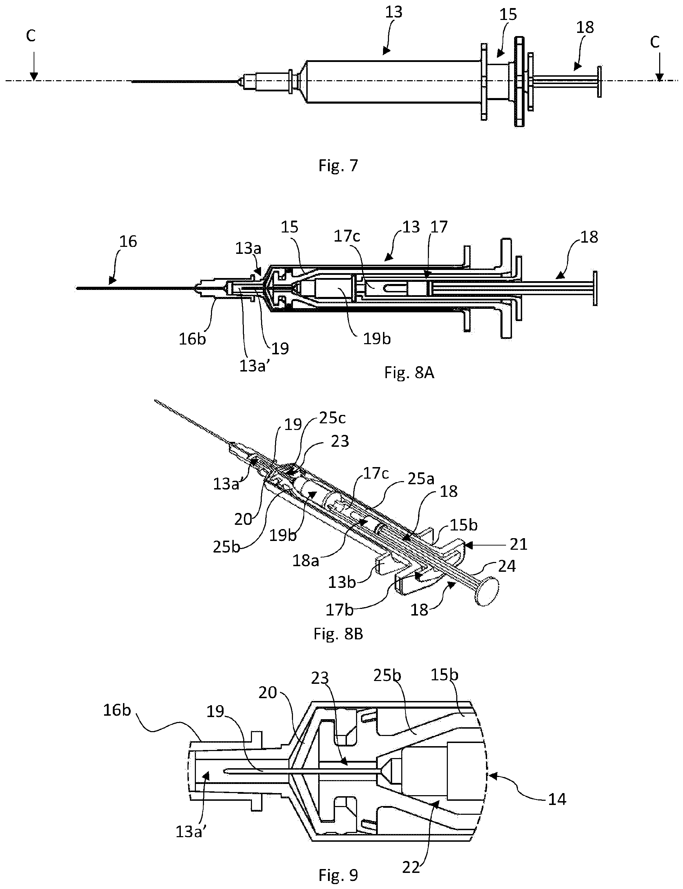

The dimensions of the internal channel 22 do not exceed excessively those of the second syringe element 14 because the internal channel 22 must act as a guide for the insertion and/or housing and/or exit of the second syringe element 14. As a matter of fact, in use, as visible for example in figure 8A, when the second syringe element 14 is housed in the first syringe element 12, in order to carry out the operation of the kit according to the present invention, the second needle 19 penetrates at least partially into the connection channel 13a' of the first end 13a of the cylindrical channel 13 or into the connection element 16b. It is therefore evincible that in order to allow such insertion correctly, the second syringe element 14 must be fittingly housed inside the internal channel 22 of the plunger 15 of the first syringe element 12. Such version is also illustrated, for example, in figures 6 and 7.

In order to penetrate into the connection channel 13a' and/or into the first connection element 16b, the second needle 19 pierces the capsule or gasket 20 of the stem 15b of the plunger 15.

As observable for example in such figures, housed inside the channel 22 are the second needle 19 (at least partially), the second connection element 19b, at least part of the cylindrical side wall 17 and thus of the piston 18 which is contained therein.

At the joint with the capsule or gasket 20, the head 15a and/or the respective end of the stem 15b of the plunger 15, in at least one version of the present invention, may have a gap or a hole 23 which allows the second needle 19 to pass and reach the capsule or gasket 20 to pierce the latter and continue the movement thereof toward the first needle 16.

This is also visible in the detail of figure 9.

The sealing of the plunger 15 is in any case ensured by the presence of the capsule or gasket 20.

When the first syringe element 12 and the second syringe element 14 of the kit 10 are assembled in this manner, it is possible to press the piston 18 of the second syringe element 14 and to let the substance contained in the space 17c flow out through the second needle 19 and then through the first needle 16. The injection of the substance contained in the second syringe element 14 (or second substance) is thus ensured exactly at the point of injection of the substance contained in the first syringe element 12 (or first substance) or at the point where the latter suctioned a possible biological liquid.

In the latter case, as mentioned above, the plunger 15 will be in a position moved away from the first end 13a of the cylindrical body 13 of the first syringe element 12 and therefore, in order to reach the first needle 16, the second needle 19 could have an adequate length (as observable in figure 16) or there could be a system, for example a suctioning system, for eliminating the liquid suctioned by the first needle 16 into the human body, so as to be able to approach the head 15a of the plunger 15 toward the first end 13a of the first syringe element 12, and thus allowing the second needle 19 to reach in proximity of the first needle 16 and carry out the injection of the substance contained in the second syringe element 14.

The piston 18 of the second syringe element 14 is provided with a stem 24. The stem 24 can be solid, possibly of the conventional type, when it has the sole purpose of passing through the space 17c and to push out the substance present in the second syringe element 14.

Alternatively, the stem 24 can be hollow and house the at least one element 30 therein, as will be better described hereinafter.

The head 18a of the piston 18 has a conformation substantially complementary to the internal one of the tip 17a of the cylindrical side wall 17 and/or of the connection channel 17a' of the tip 17a in order to press and make all the substance contained in the second syringe element 14 and/or in the space 17c flow out from the second needle 19.

As observable in the figures, and in at least one version of the invention, the plunger 15 and/or the stem 15b thereof has a substantially tubular-shaped portion 25a with a circular cross-section, with which there is associated an internally hollow cone or truncated cone portion 25b, at the head 15a. The base of the cone or truncated cone portion 25b coincides with one of the bases of the substantially tubular-shaped portion 25a. The internal channel 22 of the stem 15 is formed both by the inner space of the substantially tubular-shaped portion 25a and by the inner space of a cone or truncated cone portion 25b. The latter is particularly suitable to fittingly house the second connection element 19b of the second syringe element 14.

On the other hand, at the apical area of the cone or truncated cone portion 25b, there is present a discoidal portion 25c, which acts as a base for supporting and constraining the capsule or gasket 20.

Both the apical area of the cone or truncated cone portion 25b and the discoidal portion 25c may comprise the gap or hole 23 for the through-passing of the second needle 19.

This gap or hole 23 can be useful to facilitate the through-passing of the second needle 19 given the nature of the material the stem of the plunger 15 is made of.

As a matter of fact, the first syringe element 12 is substantially fully made (except for the first needle 16 and the capsule or gasket 20) of a plastic material resistant to deformation, in order to carry out the substance injection or suctioning function thereof. The first needle 16 and the second needle 19 are made of a metal material. The capsule or gasket 20 is made of a resistant but deformable material, and therefore suitable to allow the sliding of the plunger 15 and the possibility of being pierced by the second needle 19.

Even the second syringe element 14 is substantially fully made (except for the second needle 19 and a possible capsule or gasket) of a plastic material resistant to deformation, in order to carry out the substance injection or suctioning function thereof.

The shape of the capsule or gasket 20 is substantially complementary to the inner shape of the first end 13a of the cylindrical body 13, except, in at least one version of the invention, for the connection channel 13a'.

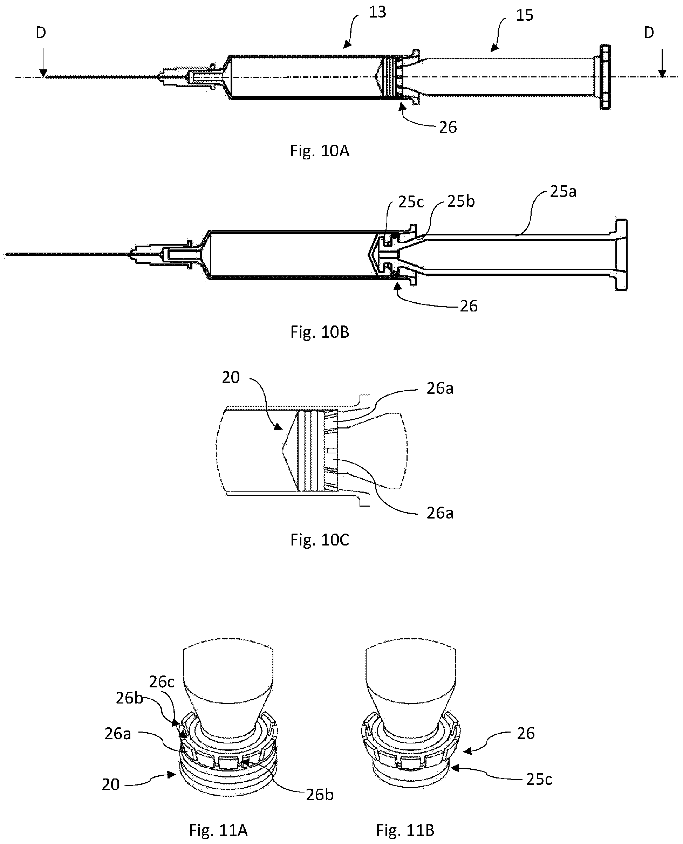

In a version of the invention, as observable in the figures 10A, 10B, 10C, 11A and 11B, the plunger 15 of the first syringe element 12 has a stop element 26. Such stop element 26 is an anti-removal element for the plunger 15, which thus cannot be erroneously removed from the cylindrical body 13 of the first syringe element 12.

As observable in the detail of figure 11A and 11B, such stop element 26 is substantially annular-shaped. It comprises a plurality of fins 26a, potentially flexible, arranged at the joint between the capsule or gasket 20 and the head 15a of the plunger 15. Such plurality of fins 26a is arranged in an annular manner around the head 15a. Each fin 26a extends from the head 15a of the plunger 15 toward the external with respect to the latter and it is faced toward the end part 21 of the plunger 15.

The plurality of fins 26a provides for that in such stop element 26 each fin is separated from the adjacent fin. In this manner, a space or gap 26b is created between one fin and the other and this allows the fins 26a to bend and/or move and/or change position should the plunger 15 be moved toward the first end 13a or toward the second end 13b of the cylindrical body 13.

In particular, should the plunger 15 be pressed toward the first end 13a of the cylindrical body 13, the fins 26a will be forced toward the stem 15b allowing the sliding of the plunger 15 in such direction; therefore, on the other hand, when the plunger 15 is pulled toward the second end 13b of the cylindrical body 13, the fins 26a will be forced toward the inner wall of the cylindrical body 13, thus carrying out the stop and/or anti-removal function carried out by the stop element 26.

Furthermore, the fact that the stop element 26 is thus shaped allows it to adapt to variable inner diameters of the cylindrical body 13 and/or of the first syringe element 12. Naturally, the diameter of the stop element 26 may vary depending on the inner diameter and/or capacity of the cylindrical body 13 of the first syringe member 12.

The larger diameter of the stop element 26 (determined by the free end of the fins 26a) is slightly larger than the diameter of the capsule or gasket 20 and/or of the head 15a of the plunger 15. In any case, given the fins 26a are flexible, the larger diameter of the stop element 26 is variable.

The flexibility of the stop element 26 may be given by the presence of the space or gap 26b between one fin and the other, or by the flexibility of the material with which the fins 26a are made, or by a combination thereof.

As observable in the figures 11A and 11B, the plurality of fins 26a is substantially rectangular parallelepiped-shaped, possibly with bevelled edges or with the free end thereof substantially rounded or curved, but other shapes can be provided. They are arranged substantially along the circumference of the capsule or gasket 20, for example to form a ring of fins 26a. In this manner, they project substantially from the capsule or gasket 20 and/or from the head 15a of the plunger 15.

Furthermore, there is present an annular hollow area 26c arranged between the plurality of fins 26a and the head 15a of the plunger 15. Such annular hollow area 26c allows the movement of the fins 26a toward the head 15a of the plunger 15, so as to allow the anti-removal function of the stop element 26.

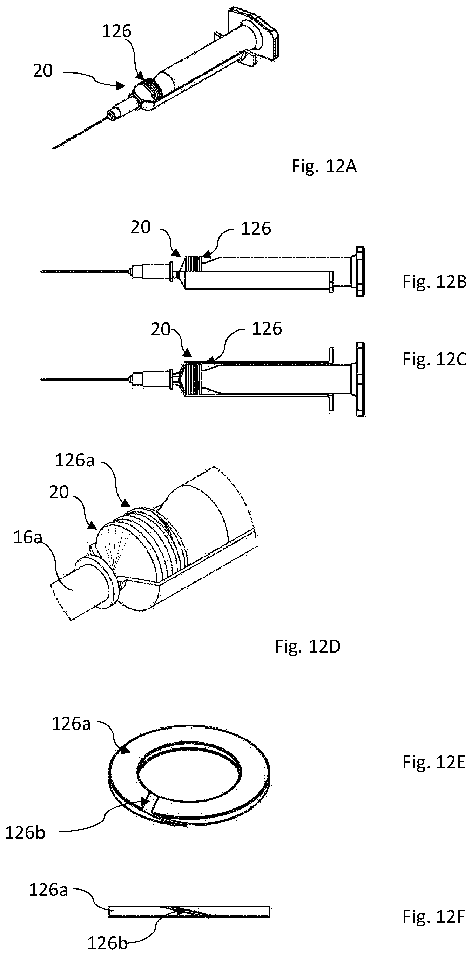

Figures 12A, 12B, 12C and 12D show a different version of the stop element 126.

It has the same function as the stop element 26 but shaped to form a ring nut 126a.

The ring nut 126a is substantially annular-shaped with a circular cross-section (visible in figures 12E and 12F). It is housed between the capsule or gasket 20 and the head 15a of the plunger 15 and/or it is fitted at the apical area of the cone or truncated cone portion 25b of the stem 15b of the plunger 15.

The ring nut 126a of the stop element 126 has a cut 126b, for example transverse and/or inclined, as shown in figure 12F.

In this manner, the ring nut 126a has an open-ring configuration and/or it can be adapted to variable inner diameters of the cylindrical body 13 of the first syringe element 12.

Naturally, the diameter of said ring nut 126a may vary according to different capacities and/or different inner diameters of the first syringe element 12.

As indicated also for the fins 26a of the stop element 26, the ring nut 126a allows the sliding of the plunger 15 toward the first end 13b of the cylindrical body 13 while, when the opposite movement of the plunger 15 away from the first end 13c occurs, the stop element 126 prevents the removal of the piston 15 from the cylindrical body 13 thanks to the ring nut 126a. As a matter of fact, the cut 126b determines a variation in the overall diameter of the ring nut 126a following the movement of the plunger 15 (a diameter which decreases while the plunger 15 is step for pushing the plunger and which increases during the step for removing the piston, actually preventing the plunger 15 from exiting from the cylindrical body 13).

In at least one version of the invention, the stop element 26, 126 is positioned distally with respect to the capsule or gasket 20, that is, arranged between the capsule or gasket 20 and the stem 15b of the plunger 15.

Therefore, as observable from the description above, in order to carry out the desired function, the second syringe element 14 is coaxial with the first syringe element 12, and/or with the internal channel 22 of the stem 15b of the plunger 15 of the first syringe element 12. Similarly, the direction of insertion of the second needle 19 at the first needle 16 occurs coaxially, so as to allow the injection of the substance contained in the second syringe element 14 exactly at the point of injection of the first needle 16 of the first syringe element 12.

The coaxiality and/or the presence at least of the hollow internal channel 22 of the stem 15b of the plunger 15 thus ensures the centring of the second needle 19.

The at least one substance present in the inner chamber which is formed in the cavity 13c between the cylindrical body 13 and the plunger 15 when the latter is at least partially housed in the cylindrical body 13 (first chamber) and/or which is formed in the space 17c between the cylindrical side wall 17 and the plunger 18, when the latter is housed at least partially in the cylindrical side wall 17 (second chamber), in at least one version of the invention, it is inserted into the respective first syringe element 12 and/or second syringe element 14 immediately before use.

For example, as a matter of fact, the surfactant must form a sort of foam that is formed before the injection thereof. Similarly, cyan acrylic glue (Loctite-Attak) or other adhesive agent polymerizes rapidly in contact with moisture and therefore it can only be loaded into the second syringe element 14 just before the use thereof.

In order to allow operation of the kit according to the present invention, in at least one version (not illustrated in the figures), the first syringe element 12 may comprise a pierceable membrane, arranged at the first end 13a of the cylindrical body 13. Such pierceable membrane is suitable to be pierced by the second needle 19 of the second syringe element 14 so that the second medical substance can be injected into the injection site of the first needle 16.

Furthermore, such pierceable membrane is suitable to allow the through-flow of a fluid (for example of the first medical substance, if present) only in the extrusion or injection direction, and not in the opposite suctioning direction, in order to prevent the second medical substance, in the injection step, from leaking in sites other than injection sites.

In at least one version of the invention, there is at least one cap for covering and protecting the first needle 16 and/or the second needle 19.

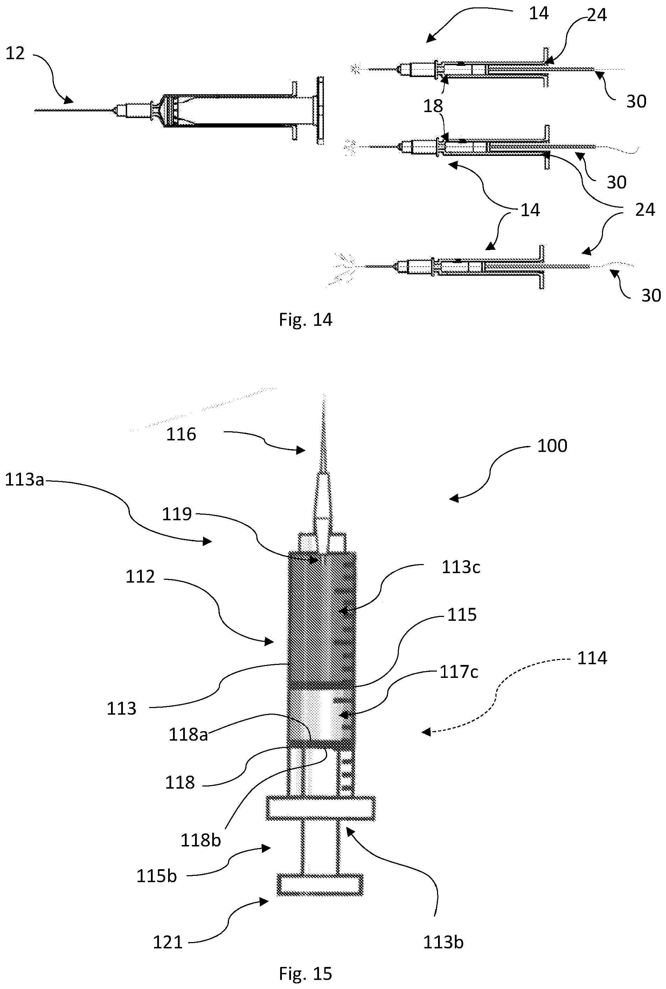

As observable in figure 14, a further version of the kit provides for that the first syringe element 12 (substantially shaped as described above) contains a substance to be injected into the human body while the second syringe element 14 comprises at least one element 30. In this case, the stem 24 of the piston 18 is hollow and it allows the insertion and housing of the element 30 (in the form of the devices listed above) thereinto. For example, figure 14 shows a second component 14 (shown in the upper position and to the right of the figure) which comprises an element 30 in the form of optical fibre and/or laser, suitable for example to heat and/or convey heat to the point of injection of the human body, while also visible is another second component 14 (shown in a central position and to the right of the figure) which comprises an element 30 in the form of a pipe for conveying steam and/or heat, or a second component 14 which comprises an element 30 in the form of a radio-frequency device or antenna (shown in a lower position and to the right).

The at least one element 30 has an extended shape and a thickness or section that is very small in size (with respect to the length thereof) so as to be able to pass through the second needle 19 and/or the first needle 16.

This version of the kit is useful, for example, when there arises the need to convey heat and/or moisture in the form of steam to the site of injection of the first substance (for example glue or an adhesive substance), or when there arises the need to dispense another sealing agent different from the first dispensed substance in such point.

In this manner, the second syringe element 14 acts as a carrier for the at least one element 30.

The at least one element 30, such as for example the laser fibre or the steam pipe illustrated in figure 14, exploits the coaxiality of the system to carry for example heat (laser or steam) exactly to the desired point.

As observable in figure 14, the at least one element 30 is inserted and/or housed at least partially inside the stem 24 of the piston 18, passing through the second needle 19 to reach near the first needle 16 (when the second syringe element 14 is housed inside the first syringe element 12) of the first syringe element 12, in which it can only be partially inserted and/or housed.

In order to do this, the second needle 19 pierces the plunger 15 and/or the capsule or gasket 20 of the first syringe element 12 as described above, so as to allow the at least one element 30 to reach in proximity of the first needle 16. Alternatively or additionally, the second needle 19 passes through the gap or hole 23 present in the plunger 15. Only after the second needle 19 has passed through the plunger 15, the at least one element 30 (in the form of fibre or pipe) is pushed toward the first end 13a of the cylindrical body 13, passing through the second needle 19 and at least partially the first needle 16. Once in proximity of the site of injection, the at least one element 30 can carry out the function thereof, such as for example that of conveying heat which, in at least one application version, may have a thrombosing property with respect to the walls of the vein to be treated.

In order to be able to reach and pass beyond the second needle 19, the at least one element 30 is capable of in turn piercing the head 18a of the piston 18 of the second syringe element 14 (for example through a piercing element arranged at the inner end of the at least one element 30), or to pass through a hole or channel (possibly initially closed by a breakable membrane which is suitably positioned and which can be perforated by the at least one element 30) which is purposely provided at the head 18a of the piston 18.

There is also described a method for the use of kit 10 and/or for the injection and/or insertion of at least one substance and/or at least one element comprising the following steps: providing a first syringe element 12 and at least a second syringe element 14, providing for at least one substance and inserting or loading at least one substance into the first syringe element 12 and/or the at least one second syringe element 14.

There are then steps of positioning the first syringe element 12 in use at an injection site of the human body, such as for example a varicose vein to be treated, injecting at least one substance (or first substance) at the injection site by the first syringe element 12 or suctioning a fluid, for example biological, from the injection site using the first syringe element 12.

Then there follows the step of positioning the plunger 15 to the end-stroke position at the first end 13a of the cylindrical body 13 of the first syringe element 12 (possibly injecting the substance contained therein into the injection site) and by a step of inserting the second syringe element 14 at the hollow internal channel 22 of the stem 15b of the plunger 15 of the first syringe element 12. Such insertion occurs along a direction coaxial to the cylindrical body 13 and/or to the first syringe element 12 and/or to the internal channel 22 of the stem 15b of the plunger 15.

This insertion occurs so that the second needle 19 of the second syringe element 14 penetrates at least partially into the first end 13a of the cylindrical body 13 and/or into the first connection element 16b of the first needle 16.

At this point, the at least one substance loaded into the second syringe element 14 (or second substance) can be loaded by means of the first needle 16 of the first syringe element 12.

Should the first syringe element 12 suction a liquid or fluid present at the injection site, firstly there will be inserted a second syringe element 14 to suction the liquid or fluid suctioned and present inside the cylindrical body 13 of the first syringe element 12. Once this step has been completed, a further second syringe element 14 will be inserted, so as to proceed with the injection of the at least one substance loaded into the latter in the injection site.

The piston 18 of the second syringe element 14 is then carried and/or pushed in the direction of the second needle 19.

Alternatively, should a biological liquid be suctioned from a site of the human body, the method provides for suctioning said liquid by means of the first syringe element 12, at least partially removing the plunger 15 from the cylindrical body and thus creating a vacuum inside the cavity 13c so as to suction the biological liquid in question in use. Then, there is provided a second syringe element 14 having a second needle 19 (as shown in fig. 16) with dimensions suitable to reach in proximity of the first needle 16 (despite the plunger 15 being in a position moved away with respect to the first end 13a of the cylindrical body 13) so as to be able to inject the substance contained in the second syringe element 14 into the suctioning site carried out by means of the first needle 16. Otherwise, there may be provided for the steps of connecting a system, for example a suctioning system, to the first syringe element 12 so as to eliminate the liquid suctioned by the first needle 16 from the first syringe element 12, thus so as to be able to approach the head 15a of the plunger 15 to the first end 13a of the first syringe element 12, and thus allow the second needle 19 to reach in proximity of the first needle 16 and carry out the injection of the substance contained in the second syringe element 14.

Naturally, in order for the kit of the present invention to operate, the cylindrical body 13 of the first syringe element 12 has a length equal to or smaller than that of the second syringe element 14, so that the piston 18 can be actuated from the external by the operator when the plunger 15 is fully inside the cylindrical body 13 and when the second syringe element 14 is positioned in the plunger 15 (as observable in the figures 6A and 6B).

During the step for inserting the second syringe element 14, the second needle 19 pierces the plunger 15 (and/or a part thereof and/or the head 15a thereof and/or the pierceable membrane) and/or the capsule or gasket 20 and/or the through-passing of the second needle 19 through the gap or hole 23 of the plunger 15.

With reference to figure 15, a kit for the subsequent injection of substances into the human body according to a further version is indicated in its entirety with reference number 100. The elements corresponding to those of the previous version will be indicated with the same reference number, increased by one hundred.

With respect to the previous version, the kit 100 requires the components to be assembled together, so as to provide a compact and fast-to-use solution. Furthermore, this is a version that is still easy and simple to handle, which can also be used by non-specialized personnel.

The kit 100 comprises a first syringe element 112. With respect to the previous version, it is as if the first syringe element 12 housed the second syringe element 14 stably and permanently therein, for example in a constrained manner. It can therefore be said that the kit 100 also comprises a second syringe element 114. As mentioned, the latter is stably and permanently constrained to the first syringe element 112.

The first syringe element 112 contains - therein - at least two substances, such as a first medical substance, such as for example a biocompatible surfactant substance, and a second medical substance, such as a biocompatible adhesive agent, of the type indicated above, or the same medical substance, but present in two different doses or densities.

In particular, the first syringe element 112 comprises, as indicated for the first version, an internally hollow cylindrical body 113, a plunger 115 (or first piston 115) and a first needle 116.

The plunger 115 hermetically slides inside the cylindrical body 113 along a longitudinal direction specifically determined by the cylindrical body 113.

In particular, the plunger 115 has a head 115a, which, in use, is suitable, when fully inserted into the cavity 113c and/or into the cylindrical body 113, to internally come into contact with the first end 113a of the cylindrical body 113.

The head 115a is faced toward the first needle 116 or toward the second needle 119. The second syringe element 114 comprises a piston 118 (or second piston 118) and a second needle 119.

The second syringe element 114 shares the cylindrical body 113 with the first syringe element 112. Therefore, unlike the second syringe element 14, the second syringe element 114 does not comprise a wall of its own, but substantially it consists of a piston 118 and a second needle 119. There is present a stem 115b for actuating the plunger 115 and the piston 118, which can be said to belong both to the first syringe element 112 and to the second syringe element 114.

Therefore, the kit 100 substantially consists of first cylindrical body 113, plunger 115, first needle 116, piston 118, second needle 119, stem 115b, obviously besides the at least one medical substance contained therein (besides the sealing and connection elements described hereinafter).

Inside the first syringe element 112 (and/or the cylindrical body 113), the presence of the plunger 115 and the piston 118 determines the definition of two spaces or chambers in which the two medical substances, such as in detail a first cavity 113c and a space 117c, can be housed.

In detail, the first cavity 113c is arranged between the plunger 115 and a first end 113a of the cylindrical body 113, while the space 117c is arranged between the plunger 115 and the piston 118.

In detail, the first medical substance is present in the first cavity 113c while the second medical substance is present in the space 117c.

Therefore, the piston 118 has a head 118a in contact with the second medical substance and a base face 118b, in use external and opposite with respect to the head 118a. Connected in use to the base face 118b is the stem 115b which is suitable to be actuated so as to allow the dispensing of the at least one medical substance or both medical substances, when both the first and second medical substances are present.

The first needle 116 and the second needle 119 are internally hollow and/or comprise respectively a first inner lumen and a second inner lumen, possibly placed in fluid communication and/or possibly coaxial.

Then there may be present a first connection element between the first needle 116 and the first end 113a of the cylindrical body 113 and/or a second connection element between the second needle 119 and the first end 113a of the cylindrical body 113 to connect, possibly removably, the first needle 116 and/or the second needle 119 to the cylindrical body 113.

In greater detail, the first needle 116 is connected externally to the first end 113a of the cylindrical body 113 while the second needle 119 is connected internally to said first end 113a. Therefore, the first needle 116 and the second needle 119 have an opposite orientation.

The cylindrical body 113 has, similarly to the first version of the invention, a second end 113b, opposite the first end 113a, and which constitutes the open end of the cylindrical body 113.

The plunger 115 slides inside the cavity 113c while the piston 118 slides inside the space 117c but actually also, in a first operative step, inside the first cavity 113c.

The first end 113a has - therein - a connection channel with the first lumen of the first needle 116 and with the second lumen of the second needle 119, as well as with the cavity 113c.

In this further version, the stem 115b may be hollow or solid.

In use, the operator acts on the stem 115b, acting in thrust thereon.

Therefore, the stem 115b is pushed toward the first needle 116, actually determining the outflow of the at least one first medical substance housed in the cavity 113c.

Since the space 117c is actually closed and filled with the at least one second medical substance, it is incompressible. When the stem 115b is actuated, it pushes the piston 118 and the space 117c, which in turn pushes the plunger 115 toward the first end 113a of the first syringe element 112. Thus, the piston 118 and the plunger 115 slide as a single body until the plunger 115 reaches in proximity of the first end 113a of the cylindrical body 113.

This is a first operative step of using the kit 100.

Once the first medical substance has been fully or substantially fully dispensed, the plunger 115 is at the inner part of the first end 113a of the first syringe element 112. In this manner, the second needle 119, which is arranged inside the first end 113a, is capable of piercing the plunger 115.

Therefore, actually while the tip of the first needle 116 is external to the first syringe element 112, for dispensing the at least one medical substance at the site of interest of the human body, the second needle 119 has a tip facing toward the internal of the cylindrical body 113. in particular, toward the plunger 115.

The length of the second needle 119 is therefore greater than the thickness of the plunger 115, so as to be able to pierce it and to be able to penetrate into the space 117c.

As the plunger 115 approaches the second needle 119, the latter begins the piercing of the plunger 115.

After piercing plunger 115, given that the space 117c is no longer a closed space, still pushing on the stem 115b, allows to move the piston 118 toward the plunger 115 and the dispensing of the second medical substance contained in the space 117c. Therefore, in such second operative step the second medical substance is dispensed exactly into the site of injection of the first medical substance, as indicated for the present invention.

The second medical substance is dispensed, through the second needle 119, by means of the first needle 116.

In this case, the stem 115b is almost fully housed inside the cavity 113c, except for the end part 121 part.

The plunger 115 and/or the head 115a of the plunger 115 may comprise a capsule or gasket, as indicated for the first version of the invention of the kit 10.

Such capsule or gasket is hermetically-sealed in the sense that it allows the sliding of the plunger 115 inside the cavity 113c of the cylindrical body 113 but, at the same time, it prevents the medical solution contained in the cavity 113c from flowing out from the side of the capsule or gasket. In which case, the second needle 119 pierces the capsule or gasket of the plunger 115.

In this version, the kit is filled with at least one substance at the factory and not by the physician.

A similar capsule or gasket may also be provided for the piston 118.

It is clear that the present invention is suitable to operate thanks to the presence of the second needle 19, 119 which is suitable to pass through the plunger 15, 115 in use.

In a version of the invention, the plunger 115 may comprise a stop element, as described for the kit 10 of the present invention.

It has thus been observed that the present invention may adapt to specific surgical requirements, by providing a kit that is easy and practical to use, which allows precision at the implantation site even for injections subsequent to the first, at the same injection site.

Characteristics described for an embodiment or variant may also be present in other embodiments or variants, without departing from the scope of protection outlined by the attached claims.

The kit 10, 100 according to the present invention is susceptible to numerous modifications and variants without departing from the scope of protection of the claims that follow.

## Claims

1. Kit (10, 100) for the injection of at least one substance in a fluid or liquid state and/or for the insertion of at least one substance and at least one element (30) in an injection or insertion site of the human body comprising a first syringe element (12, 112) and at least one second syringe element (14, 114), wherein said first syringe element (12, 112) comprises an internally hollow cylindrical body (13, 113), a plunger (15, 115) and a first needle (16, 116), in which said plunger (15, 115) is adapted in use to slide inside said cylindrical body (13, 113), said plunger (15, 115) comprising a head (15a, 115a) which faces, in use, said first needle (16, 116) and a stem (15b, 115b) which is internally hollow and comprises in its inner part an internal channel (22), wherein said at least one second syringe element (14 , 114) comprises a piston (18, 118) and a second needle (19, 119), wherein at least one substance and/or said at least one element (30) is inserted inside said cylindrical body (13, 113) and/or inside said second syringe element (14), **characterized in that** said internal channel (22) is shaped in such a way as to allow the housing of said second syringe element (14) inside it, said second needle (19, 119) being adapted in use to pass through said plunger (15, 115) and to pierce said plunger (15, 115) so as to allow the at least one element (30) to reach in proximity of the first needle (16).

2. Kit (10, 100) according to claim 1, wherein said internal channel (22) is coaxial with said stem (15b) and/or with said first syringe element (12) and/or wherein said internal channel (22) has a conformation corresponding and/or complementary to that of said second syringe element (14), but slightly larger to allow the, possibly removable, housing of said second syringe element (14).

3. Kit (10, 100) according to claim 1, wherein said plunger (15) and/or said stem (15b) comprises a substantially tubular shaped portion (25a) and, at said head (15a), a cone or truncated cone portion (25b), in which said channel (22) of said stem (15) involves both said substantially tubular shaped portion (25a) and said cone or truncated cone portion (25b).

4. Kit (10, 100) according to the preceding claim, wherein at an apical area of said cone or truncated cone portion (25b), facing said first needle (16), there is a discoidal portion (25c), which acts as a base of support and constraint for said capsule or gasket (20).

5. Kit (10, 100) according to any one of the preceding claims, wherein said first needle (16, 116) and said second needle (19, 119) are internally hollow and/or comprise respectively a first internal lumen (16a) and a second internal lumen (19a) through which the at least a medical substance can in use flow.

6. Kit (10, 100) according to any one of the preceding claims, wherein said cylindrical body (13, 113) comprises a first end (13a, 113a) to which said first needle (16, 116) is constrained, possibly in a removable way, a cavity (13c, 113c) inside said cylindrical body (13, 113) and a second end (13b, 113b), opposite to said first end (13a, 113a), wherein said second end (13b, 113b) is open and for access to said cavity (13c, 113c).

7. Kit (10, 100) according to any one of the preceding claims, wherein said second syringe element (14) comprises a cylindrical side wall (17), internally hollow, comprising a tip (17a), to which said second needle (19) is constrained, possibly in a removable way, an internal space (17c) to said cylindrical side wall (17) possibly for housing said at least one substance, and a distal end (17b), opposite to said tip (17a), wherein said distal end (17b) is open and for access to said space (17c), in which said piston (18) is adapted in use to slide inside said cylindrical side wall (17) and/or in which said second piston (118) is adapted in use to slide inside said first cavity (113c) of said first syringe element (112).

8. Kit (10, 100) according to any one of the preceding claims, wherein said plunger (15, 115) and/or said head (15a, 115a) of said plunger (15, 115) comprises a capsule or gasket (20), wherein said capsule or gasket (20) is made of rubber and/or has a substantially conical conformation or corresponding to the internal zone of said first end (13a, 113a) of said cylindrical body (13, 113), and/or in which said capsule or gasket (20) is adapted in use to be perforated by said second needle (19, 119).

9. Kit (10, 100) according to any one of the preceding claims, wherein, at said capsule or gasket (20), said head (15a) and/or said plunger (15) and/or said discoidal portion (25c) and/or said cone or truncated cone portion (25b) has an opening or hole (23) for the passage in use of said second needle (19).

10. Kit (10, 100) according to any one of the preceding claims, comprising a first connection element (16b) between said first needle (16, 116) and said cylindrical body (13, 113) to connect to the latter, possibly in a removable way, said first needle (16, 116) and/or a second connection element (19b) between said second needle (19, 119) and said cylindrical body (13, 113) or said cylindrical side wall (17) to connect to the latter, possibly in a removable way, said second needle (19, 119) and/or in which said plunger (15) comprises a n opening or a hole (23) at said head (15a), in which said first connection element (16b) is adapted in use to house at least partially said second needle (19).

11. Kit (10, 100) according to any one of the preceding claims, wherein said at least one substance is a medical substance such as a biocompatible surfactant substance, such as for example a surfactant sclerosing drug such as sodium tetradecyl sulfate known by the trade name Fibro-Vein^{®} or Trombovar^{®} or oxy-polyethoxy dodecane known by the trade name Atossisclerol^{®} or Sotradecol^{®}, etc., a biocompatible gluing agent, for example based on cyanoacrylate, such as for example the one known by the trade name Loctite-Attak, and/or a biocompatible cementing element.

12. Kit (10, 100) according to any one of the preceding claims, comprising a stop element (26, 126), of substantially annular conformation, adapted in use to at least partially prevent the extraction of said plunger (15, 115) from said cylindrical body (13, 113), wherein said stop element (26, 126) is placed at said capsule or gasket (20) and/or said head (15a, 115a) of said plunger (15, 115).

13. Kit (10, 100) according claim 12, wherein said stop element (26) comprises a plurality of fins (26a), flexible if desired, arranged annularly around said head (15a, 115a) and/or wherein each fin (26a) departs from said head (15a, 115a) outwards with respect to said plunger (15, 115) and/or facing in use an end part (21, 121) of said plunger (15, 115) and/or said second end (13b, 113b) of said cylindrical body (13, 113 ) and/or wherein each fin (26a) is separated from each adjacent fin (26a) by a space or gap (26b) between one fin and the other, so that each fin (26a) of said plurality flexes and/or moves and/or changes position if said plunger (15, 115) is moved towards said first end (13a, 113a) or towards said second end (13b, 113b) of said cylindrical body (13, 113 ).

14. Kit (10, 100) according to claim 12 or 13, wherein said fins (26a) have a free end and said stop element (26) has a larger diameter at said free end which is slightly greater than the diameter of said capsule or gasket (20) and/or of said head (15a, 115a) of said plunger (15, 115) and/or wherein there is an annular hollow area (26c) located between said plurality of fins (26a) and said head (15a, 115a) of said plunger (15, 115) for allowing the bending and/or the movement of said fins (26a) towards said head (15a, 115a).

15. Kit (10, 100) according to claim 12, wherein said stop element (126) has a substantially annular ring nut (126a) conformation with cylindrical section and/or has a ring nut conformation (126a) comprising a cut (126b), for example transverse and/or inclined, so that said ring nut (126a) is adaptable with variable internal diameters of said cylindrical body (13, 113) of said first syringe element (12, 112).

16. Kit (10, 100) according to any one of the preceding claims, wherein said at least one substance is housed in a first internal chamber formed in said cavity (13c, 113c) between cylindrical body (13, 113) and plunger (15, 115) when the latter is at least partially housed inside said cylindrical body (13, 113) of said first syringe element (12, 112) and/or in a second internal chamber formed in said space (17c) between cylindrical side wall (17) and piston (18), when the latter is at least partially housed inside said cylindrical side wall (17) of said second syringe element (14) and/or in a second internal chamber formed between said plunger (115) and said piston (118) when both are housed inside of said cylindrical body (113) of said first syringe element (112).

17. Kit (10, 100) according to any one of the preceding claims, wherein said second syringe element (14) comprises at least one element (30), in the form of an optical fiber device, a laser fiber device, a device capable of transporting and/or delivering steam or other suitable gas, a radiofrequency device or antenna, for example having a tubular conformation, a device capable of generating heat and/or radiation, for example in the infrared spectrum, or microwaves, etc., wherein said at least one element (30) has an elongated conformation and is able to be housed inside said piston (18), said second needle (19) and to reach near or be at least partially housed in said first needle (16) and/or wherein said at least one element (30) possibly comprises a perforating element placed at an internal part thereof to perforate said piston (18) and/or wherein said at least one element (30) is able to be housed in said hollow stem ( 24) of said piston (18) and to be housed or pushed into a channel present in said head (18a) of said piston (18).

18. Kit (10, 100) according to any one of the preceding claims, wherein said first syringe element (12) comprises a pierceable membrane, located at said first end (13a) of said cylindrical body (13), in which said pierceable membrane is adapted to be pierced by said second needle (19) of said second syringe element (14) so that said second needle comes in proximity of said first needle (16).

19. Kit (10, 100) according to any one of the preceding claims, wherein said first needle (16, 116) is facing in use the outside of said first syringe element (12, 112) and/or towards the injection site while said second needle (119) is facing in use said first cavity (113c) of said cylindrical body (113), in which said first needle (16, 116) and said second needle (119) are coaxial.

## Patentansprüche

1. Kit (10, 100) zur Injektion mindestens einer Substanz in fluidem oder flüssigem Zustand und/oder zum Einführen mindestens einer Substanz und mindestens eines Elements (30) in eine Injektions- oder Einführstelle des menschlichen Körpers, umfassend ein erstes Spritzenelement (12, 112) und mindestens ein zweites Spritzenelement (14, 114), wobei das erste Spritzenelement (12, 112) einen innen hohlen zylindrischen Körper (13, 113), einen Stößel (15, 115) und eine erste Nadel (16, 116) umfasst, in dem der Stößel (15, 115) im Gebrauch dazu angepasst ist, innerhalb des zylindrischen Körpers (13, 113) zu gleiten, wobei der Stößel (15, 115) einen Kopf (15a, 115a), der im Gebrauch der ersten Nadel (16, 116) zugewandt ist, und einen Schaft (15b, 115b) umfasst, der innen hohl ist und in seinem inneren Teil einen inneren Kanal (22) umfasst, wobei das mindestens eine zweite Spritzenelement (14, 114) einen Kolben (18, 118) und eine zweite Nadel (19, 119) umfasst, wobei mindestens eine Substanz und/oder das mindestens eine Element (30) in den zylindrischen Körper (13, 113) und/oder in das zweite Spritzenelement (14) hineingeführt wird, **dadurch gekennzeichnet, dass** der innere Kanal (22) so geformt ist, dass die Aufnahme des zweiten Spritzenelements (14) in ihm ermöglicht wird, wobei die zweite Nadel (19, 119) im Gebrauch dazu angepasst ist, durch den Stößel (15, 115) hindurchzutreten und den Stößel (15, 115) zu durchstechen, so dass das mindestens eine Element (30) in die Nähe der ersten Nadel (16) gelangen kann.

2. Kit (10, 100) nach Anspruch 1, wobei der innere Kanal (22) koaxial zu dem Schaft (15b) und/oder zu dem ersten Spritzenelement (12) ist und/oder wobei der innere Kanal (22) eine Gestalt aufweist, die derjenigen des zweiten Spritzenelements (14) entspricht und/oder komplementär ist, jedoch etwas größer ist, um die, möglicherweise entfernbare, Aufnahme des zweiten Spritzenelements (14) zu ermöglichen.

3. Kit (10, 100) nach Anspruch 1, wobei der Stößel (15) und/oder der Schaft (15b) einen im Wesentlichen rohrförmigen Abschnitt (25a) und an dem Kopf (15a) einen Kegel- oder Kegelstumpfabschnitt (25b) umfasst, wobei der Kanal (22) des Schafts (15) sowohl den im Wesentlichen rohrförmigen Abschnitt (25a) als auch den Kegel- oder Kegelstumpfabschnitt (25b) beinhaltet.

4. Kit (10, 100) nach dem vorhergehenden Anspruch, wobei an einem apikalen Bereich des Kegel- oder Kegelstumpfabschnitts (25b), der der ersten Nadel (16) zugewandt ist, ein scheibenförmiger Abschnitt (25c) vorhanden ist, der als Basis der Abstützung und Befestigung für die Kapsel oder Dichtung (20) dient.

5. Kit (10, 100) nach einem der vorhergehenden Ansprüche, wobei die erste Nadel (16, 116) und die zweite Nadel (19, 119) innen hohl sind und/oder jeweils ein erstes inneres Lumen (16a) und ein zweites inneres Lumen (19a) umfassen, durch die die mindestens eine medizinische Substanz im Gebrauch fließen kann.

6. Kit (10, 100) nach einem der vorhergehenden Ansprüche, wobei der zylindrische Körper (13, 113) ein erstes Ende (13a, 113a), an das die erste Nadel (16, 116), möglicherweise auf entfernbare Weise, befestigt ist, einen Hohlraum (13c, 113c) innerhalb des zylindrischen Körpers (13, 113) und ein zweites Ende (13b, 113b) gegenüber dem ersten Ende (13a, 113a) umfasst, wobei das zweite Ende (13b, 113b) offen ist und Zugang zu dem Hohlraum (13c, 113c) bietet.

7. Kit (10, 100) nach einem der vorhergehenden Ansprüche, wobei das zweite Spritzenelement (14) eine zylindrische Seitenwand (17), die innen hohl ist und eine Spitze (17a) umfasst, an die die zweite Nadel (19), möglicherweise auf entfernbare Weise, befestigt ist, einen Innenraum (17c) an der zylindrischen Seitenwand (17), möglicherweise zur Aufnahme der mindestens einen Substanz, und ein distales Ende (17b) gegenüber der Spitze (17a) umfasst, wobei das distale Ende (17b) offen ist und Zugang zu dem Raum (17c) bietet, in dem der Kolben (18) im Gebrauch dazu angepasst ist, innerhalb der zylindrischen Seitenwand (17) zu gleiten, und/oder in dem der zweite Kolben (118) im Gebrauch dazu angepasst ist, innerhalb des ersten Hohlraums (113c) des ersten Spritzenelements (112) zu gleiten.

8. Kit (10, 100) nach einem der vorhergehenden Ansprüche, wobei der Stößel (15, 115) und/oder der Kopf (15a, 115a) des Stößels (15, 115) eine Kapsel oder Dichtung (20) umfasst, wobei die Kapsel oder Dichtung (20) aus Gummi hergestellt ist und/oder eine Gestalt aufweist, die im Wesentlichen konisch ist oder der inneren Zone des ersten Endes (13a, 113a) des zylindrischen Körpers (13, 113) entspricht und/oder wobei die Kapsel oder Dichtung (20) im Gebrauch dazu angepasst ist, von der zweiten Nadel (19, 119) perforiert zu werden.

9. Kit (10, 100) nach einem der vorhergehenden Ansprüche, wobei an der Kapsel oder Dichtung (20) der Kopf (15a) und/oder der Stößel (15) und/oder der scheibenförmige Abschnitt (25c) und/oder der Kegel- oder Kegelstumpfabschnitt (25b) eine Öffnung oder ein Loch (23) für den Durchgang im Gebrauch der zweiten Nadel (19) aufweist.

10. Kit (10, 100) nach einem der vorhergehenden Ansprüche, umfassend ein erstes Verbindungselement (16b) zwischen der ersten Nadel (16, 116) und dem zylindrischen Körper (13, 113), um die erste Nadel (16, 116) mit dem letzteren, möglicherweise auf entfernbare Weise, zu verbinden und/oder ein zweites Verbindungselement (19b) zwischen der zweiten Nadel (19, 119) und dem zylindrischen Körper (13, 113) oder der zylindrischen Seitenwand (17), um die zweite Nadel (19, 119) mit der letzteren, möglicherweise auf entfernbare Weise, zu verbinden und/oder wobei der Stößel (15) eine Öffnung oder ein Loch (23) an dem Kopf (15a) umfasst, wobei das erste Verbindungselement (16b) im Gebrauch dazu angepasst ist, die zweite Nadel (19) zumindest teilweise aufzunehmen.

11. Kit (10, 100) nach einem der vorhergehenden Ansprüche, wobei es sich bei der mindestens einen Substanz um eine medizinische Substanz wie eine biokompatible oberflächenaktive Substanz, wie beispielsweise ein oberflächenaktives sklerosierendes Medikament wie Natriumtetradecylsulfat, das unter dem Handelsnamen Fibro-Vein^{®} oder Trombovar^{®} bekannt ist, oder Oxy-Polyethoxy-Dodecan, das unter dem Handelsnamen Atossisclerol^{®} oder Sotradecol^{®} bekannt ist, usw., ein biokompatibles Klebemittel, beispielsweise auf Basis von Cyanoacrylat, wie beispielsweise das unter dem Handelsnamen Loctite-Attak bekannte, und/oder ein biokompatibles Zementierelement handelt.

12. Kit (10, 100) nach einem der vorhergehenden Ansprüche, umfassend ein Anschlagelement (26, 126) mit im Wesentlichen ringförmiger Gestalt, das im Gebrauch dazu angepasst ist, das Herausziehen des Stößels (15, 115) aus dem zylindrischen Körper (13, 113) zumindest teilweise zu verhindern, wobei das Anschlagelement (26, 126) an der Kapsel oder Dichtung (20) und/oder dem Kopf (15a, 115a) des Stößels (15, 115) angeordnet ist.

13. Kit (10, 100) nach Anspruch 12, wobei das Anschlagelement (26) eine Vielzahl von gegebenenfalls flexiblen Rippen (26a) umfasst, die ringförmig um den Kopf (15a, 115a) angeordnet sind und/oder wobei sich eine jede Rippe (26a) von dem Kopf (15a, 115a) nach außen in Bezug auf den Stößel (15, 115) erstreckt und/oder im Gebrauch einem Endteil (21, 121) des Stößels (15, 115) und/oder dem zweiten Ende (13b, 113b) des zylindrischen Körpers (13, 113) zugewandt ist und/oder wobei eine jede Rippe (26a) von einer jeden benachbarten Rippe (26a) durch einen Raum oder Spalt (26b) zwischen einer Rippe und der anderen getrennt ist, so dass sich eine jede Rippe (26a) der Vielzahl biegt und/oder bewegt und/oder ihre Position ändert, wenn der Stößel (15, 115) in Richtung des ersten Endes (13a, 113a) oder in Richtung des zweiten Endes (13b, 113b) des zylindrischen Körpers (13, 113) bewegt wird.

14. Kit (10, 100) nach Anspruch 12 oder 13, wobei die Rippen (26a) ein freies Ende aufweisen und das Anschlagelement (26) an dem freien Ende einen größeren Durchmesser aufweist, der etwas größer ist als der Durchmesser der Kapsel oder Dichtung (20) und/oder des Kopfes (15a, 115a) des Stößels (15, 115) und/oder wobei ein ringförmiger hohler Bereich (26c) vorhanden ist, der zwischen der Vielzahl von Rippen (26a) und dem Kopf (15a, 115a) des Stößels (15, 115) liegt, um das Biegen und/oder die Bewegung der Rippen (26a) in Richtung des Kopfes (15a, 115a) zu ermöglichen.

15. Kit (10, 100) nach Anspruch 12, wobei das Anschlagelement (126) im Wesentlichen wie eine ringförmige Ringmutter (126a) mit zylindrischem Abschnitt gestaltet ist und/oder wie eine Ringmutter (126a) gestaltet ist, umfassend einen Schnitt (126b), beispielsweise quer und/oder geneigt, so dass die Ringmutter (126a) an variable Innendurchmesser des zylindrischen Körpers (13, 113) des ersten Spritzenelements (12, 112) anpassbar ist.

16. Kit (10, 100) nach einem der vorhergehenden Ansprüche, wobei die mindestens eine Substanz in einer ersten inneren Kammer, die in dem Hohlraum (13c, 113c) zwischen dem zylindrischen Körper (13, 113) und dem Stößel (15, 115) ausgebildet ist, wenn letzterer zumindest teilweise innerhalb des zylindrischen Körpers (13, 113) des ersten Spritzenelements (12, 112) aufgenommen ist, und/oder in einer zweiten inneren Kammer, die in dem Raum (17c) zwischen der zylindrischen Seitenwand (17) und dem Kolben (18) ausgebildet ist, wenn letzterer zumindest teilweise innerhalb der zylindrischen Seitenwand (17) des zweiten Spritzenelements (14) aufgenommen ist, und/oder in einer zweiten inneren Kammer aufgenommen ist, die zwischen dem Stößel (115) und dem Kolben (118) ausgebildet ist, wenn beide innerhalb des zylindrischen Körpers (113) des ersten Spritzenelements (112) aufgenommen sind.

17. Kit (10, 100) nach einem der vorhergehenden Ansprüche, wobei das zweite Spritzenelement (14) mindestens ein Element (30) in Form einer optischen Faservorrichtung, einer Laserfaservorrichtung, einer Vorrichtung, die Dampf oder ein anderes geeignetes Gas transportieren und/oder abgeben kann, einer Hochfrequenzvorrichtung oder -antenne, beispielsweise mit rohrförmiger Gestalt, einer Vorrichtung, die Wärme und/oder Strahlung, beispielsweise im Infrarotspektrum, oder Mikrowellen usw. erzeugen kann, umfasst, wobei das mindestens eine Element (30) eine längliche Gestalt aufweist und in der Lage ist, innerhalb des Kolbens (18), der zweiten Nadel (19) aufgenommen zu werden und nahe an der ersten Nadel (16) zu gelangen oder zumindest teilweise darin aufgenommen zu werden und/oder wobei das mindestens eine Element (30) möglicherweise ein Perforationselement umfasst, das an einem inneren Teil davon angeordnet ist, um den Kolben (18) zu perforieren und/oder wobei das mindestens eine Element (30) in dem hohlen Schaft (24) des Kolbens (18) aufgenommen werden kann und in einen im Kopf (18a) des Kolbens (18) vorhandenen Kanal aufgenommen oder geschoben werden kann.

18. Kit (10, 100) nach einem der vorhergehenden Ansprüche, wobei das erste Spritzenelement (12) eine durchstechbare Membran umfasst, die an dem ersten Ende (13a) des zylindrischen Körpers (13) liegt, wobei die durchstechbare Membran dazu angepasst ist, von der zweiten Nadel (19) des zweiten Spritzenelements (14) durchstochen zu werden, so dass die zweite Nadel in die Nähe der ersten Nadel (16) kommt.

19. Kit (10, 100) nach einem der vorhergehenden Ansprüche, wobei die erste Nadel (16, 116) im Gebrauch der Außenseite des ersten Spritzenelements (12, 112) und/oder der Injektionsstelle zugewandt ist, während die zweite Nadel (119) im Gebrauch dem ersten Hohlraum (113c) des zylindrischen Körpers (113) zugewandt ist, wobei die erste Nadel (16, 116) und die zweite Nadel (119) koaxial sind.

## Revendications

1. Kit (10, 100) pour l'injection d'au moins une substance à l'état fluide ou liquide et/ou pour l'insertion d'au moins une substance et d'au moins un élément (30) dans un site d'injection ou d'insertion du corps humain, comprenant un premier élément de seringue (12, 112) et au moins un second élément de seringue (14, 114), dans lequel ledit premier élément de seringue (12, 112) comprend un corps cylindrique intérieurement creux (13, 113), un plongeur (15, 115) et une première aiguille (16, 116), dans lequel ledit plongeur (15, 115) est adapté, lors de l'utilisation, pour coulisser à l'intérieur dudit corps cylindrique (13, 113), ledit plongeur (15, 115) comprenant une tête (15a, 115a) qui fait face, lors de l'utilisation, à ladite première aiguille (16, 116) et une tige (15b, 115b) qui est creuse intérieurement et comprend dans sa partie interne un canal interne (22), dans lequel ledit au moins un second élément de seringue (14, 114) comprend un piston (18, 118) et une seconde aiguille (19, 119), dans lequel au moins une substance et/ou ledit au moins un élément (30) est inséré(e) à l'intérieur dudit corps cylindrique (13, 113) et/ou à l'intérieur dudit second élément de seringue (14), **caractérisé en ce que** ledit canal interne (22) est façonné de manière à permettre le logement dudit second élément de seringue (14) à l'intérieur de celui-ci, ladite seconde aiguille (19, 119) étant adaptée, lors de l'utilisation, pour passer à travers ledit plongeur (15, 115) et pour percer ledit plongeur (15, 115) de manière à permettre à l'au moins un élément (30) de parvenir à proximité de la première aiguille (16).

2. Kit (10, 100) selon la revendication 1, dans lequel ledit canal interne (22) est coaxial avec ladite tige (15b) et/ou avec ledit premier élément de seringue (12) et/ou dans lequel ledit canal interne (22) présente une conformation correspondant et/ou complémentaire à celle dudit second élément de seringue (14), mais légèrement plus grande pour permettre le logement, éventuellement amovible, dudit second élément de seringue (14).

3. Kit (10, 100) selon la revendication 1, dans lequel ledit plongeur (15) et/ou ladite tige (15b) comprend une partie de forme sensiblement tubulaire (25a) et, au niveau de ladite tête (15a), une partie conique ou tronconique (25b), dans lequel ledit canal (22) de ladite tige (15) implique à la fois ladite partie de forme sensiblement tubulaire (25a) et ladite partie conique ou tronconique (25b) .

4. Kit (10, 100) selon la revendication précédente, dans lequel, au niveau d'une zone apicale de ladite partie conique ou tronconique (25b), faisant face à ladite première aiguille (16), se trouve une partie discoïdale (25c), qui sert de base de support et de contrainte pour ladite capsule ou ledit joint (20).

5. Kit (10, 100) selon l'une quelconque des revendications précédentes, dans lequel ladite première aiguille (16, 116) et ladite seconde aiguille (19, 119) sont creuses intérieurement et/ou comprennent respectivement une première lumière interne (16a) et une seconde lumière interne (19a) à travers lesquelles l'au moins une substance médicale peut s'écouler lors de l'utilisation.

6. Kit (10, 100) selon l'une quelconque des revendications précédentes, dans lequel ledit corps cylindrique (13, 113) comprend une première extrémité (13a, 113a) à laquelle ladite première aiguille (16, 116) est contrainte, éventuellement de manière amovible, une cavité (13c, 113c) à l'intérieur dudit corps cylindrique (13, 113) et une seconde extrémité (13b, 113b), opposée à ladite première extrémité (13a, 113a), dans lequel ladite seconde extrémité (13b, 113b) est ouverte et permet d'accéder à ladite cavité (13c, 113c).

7. Kit (10, 100) selon l'une quelconque des revendications précédentes, dans lequel ledit second élément de seringue (14) comprend une paroi latérale cylindrique (17), creuse intérieurement, comprenant une pointe (17a), à laquelle ladite seconde aiguille (19) est contrainte, éventuellement de manière amovible, un espace interne (17c) à ladite paroi latérale cylindrique (17) éventuellement destiné à loger ladite au moins une substance, et une extrémité distale (17b), opposée à ladite pointe (17a), dans lequel ladite extrémité distale (17b) est ouverte et permet d'accéder audit espace (17c), dans lequel ledit piston (18) est adapté, lors de l'utilisation, pour coulisser à l'intérieur de ladite paroi latérale cylindrique (17) et/ou dans lequel ledit second piston (118) est adapté, lors de l'utilisation, pour coulisser à l'intérieur de ladite première cavité (113c) dudit premier élément de seringue (112).

8. Kit (10, 100) selon l'une quelconque des revendications précédentes, dans lequel ledit plongeur (15, 115) et/ou ladite tête (15a, 115a) dudit plongeur (15, 115) comprend une capsule ou un joint (20), dans lequel ladite capsule ou ledit joint (20) est en caoutchouc et/ou présente une conformation sensiblement conique ou correspondant à la zone interne de ladite première extrémité (13a, 113a) dudit corps cylindrique (13, 113), et/ou dans lequel ladite capsule ou ledit joint (20) est adapté(e), lors de l'utilisation, pour être perforé(e) par ladite seconde aiguille (19, 119).

9. Kit (10, 100) selon l'une quelconque des revendications précédentes, dans lequel, au niveau de ladite capsule ou dudit joint (20), ladite tête (15a) et/ou ledit plongeur (15) et/ou ladite partie discoïdale (25c) et/ou ladite partie conique ou tronconique (25b) comporte(ent) une ouverture ou un trou (23) pour le passage, lors de l'utilisation, de ladite seconde aiguille (19).

10. Kit (10, 100) selon l'une quelconque des revendications précédentes, comprenant un premier élément de liaison (16b) entre ladite première aiguille (16, 116) et ledit corps cylindrique (13, 113) pour relier à ce dernier, éventuellement de manière amovible, ladite première aiguille (16, 116) et/ou un second élément de liaison (19b) entre ladite seconde aiguille (19, 119) et ledit corps cylindrique (13, 113) ou ladite paroi latérale cylindrique (17) pour relier à cette dernière, éventuellement de manière amovible, ladite seconde aiguille (19, 119) et/ou dans lequel ledit plongeur (15) comprend une ouverture ou un trou (23) au niveau de ladite tête (15a), dans lequel ledit premier élément de liaison (16b) est adapté, lors de l'utilisation, pour loger au moins partiellement ladite seconde aiguille (19).

11. Kit (10, 100) selon l'une quelconque des revendications précédentes, dans lequel ladite au moins une substance est une substance médicale telle qu'une substance tensioactive biocompatible, telle que par exemple un médicament sclérosant tensioactif tel que le sulfate de tétradécyle de sodium connu sous le nom commercial Fibro-Vein^{®} ou Trombovar^{®} ou l'oxy-polyéthoxy dodécane connu sous le nom commercial Atossisclerol^{®} ou Sotradecol^{®}, etc., un agent de collage biocompatible, par exemple à base de cyanoacrylate, tel que par exemple celui connu sous le nom commercial Loctite-Attak, et/ou un élément de cimentation biocompatible.

12. Kit (10, 100) selon l'une quelconque des revendications précédentes, comprenant un élément d'arrêt (26, 126), de conformation sensiblement annulaire, adapté, lors de l'utilisation, pour empêcher au moins partiellement l'extraction dudit plongeur (15, 115) dudit corps cylindrique (13, 113), dans lequel ledit élément d'arrêt (26, 126) est placé au niveau de ladite capsule ou dudit joint (20) et/ou de ladite tête (15a, 115a) dudit plongeur (15, 115).

13. Kit (10, 100) selon la revendication 12, dans lequel ledit élément d'arrêt (26) comprend une pluralité d'ailettes (26a), flexibles le cas échéant, disposées de manière annulaire autour de ladite tête (15a, 115a) et/ou dans lequel chaque ailette (26a) s'éloigne de ladite tête (15a, 115a) vers l'extérieur par rapport audit plongeur (15, 115) et/ou faisant face, lors de l'utilisation, à une partie d'extrémité (21, 121) dudit plongeur (15, 115) et/ou à ladite seconde extrémité (13b, 113b) dudit corps cylindrique (13, 113) et/ou dans lequel chaque ailette (26a) est séparée de chaque ailette adjacente (26a) par un espace ou un intervalle (26b) entre une ailette et l'autre, de sorte que chaque ailette (26a) de ladite pluralité fléchit et/ou se déplace et/ou change de position si ledit plongeur (15, 115) est déplacé vers ladite première extrémité (13a, 113a) ou vers ladite seconde extrémité (13b, 113b) dudit corps cylindrique (13, 113).

14. Kit (10, 100) selon la revendication 12 ou 13, dans lequel lesdites ailettes (26a) comportent une extrémité libre et ledit élément d'arrêt (26) a un diamètre plus grand au niveau de ladite extrémité libre qui est légèrement supérieur au diamètre de ladite capsule ou dudit joint (20) et/ou de ladite tête (15a, 115a) dudit plongeur (15, 115) et/ou dans lequel il se trouve une zone creuse annulaire (26c) située entre ladite pluralité d'ailettes (26a) et ladite tête (15a, 115a) dudit plongeur (15, 115) pour permettre la flexion et/ou le mouvement desdites ailettes (26a) vers ladite tête (15a, 115a).

15. Kit (10, 100) selon la revendication 12, dans lequel ledit élément d'arrêt (126) a une conformation d'écrou à œil (126a) sensiblement annulaire avec une section cylindrique et/ou a une conformation d'écrou à œil (126a) comprenant une découpe (126b), par exemple transversale et/ou inclinée, de sorte que ledit écrou à œil (126a) est adaptable à des diamètres internes variables dudit corps cylindrique (13, 113) dudit premier élément de seringue (12, 112).

16. Kit (10, 100) selon l'une quelconque des revendications précédentes, dans lequel ladite au moins une substance est logée dans une première chambre interne formée dans ladite cavité (13c, 113c) entre le corps cylindrique (13, 113) et le plongeur (15, 115) lorsque ce dernier est au moins partiellement logé à l'intérieur dudit corps cylindrique (13, 113) dudit premier élément de seringue (12, 112) et/ou dans une seconde chambre interne formée dans ledit espace (17c) entre la paroi latérale cylindrique (17) et le piston (18), lorsque ce dernier est au moins partiellement logé à l'intérieur de ladite paroi latérale cylindrique (17) dudit second élément de seringue (14) et/ou dans une seconde chambre interne formée entre ledit plongeur (115) et ledit piston (118) lorsque les deux sont logés à l'intérieur dudit corps cylindrique (113) dudit premier élément de seringue (112).

17. Kit (10, 100) selon l'une quelconque des revendications précédentes, dans lequel ledit second élément de seringue (14) comprend au moins un élément (30), sous la forme d'un dispositif à fibre optique, d'un dispositif à fibre laser, d'un dispositif capable de transporter et/ou de délivrer de la vapeur ou un autre gaz approprié, d'un dispositif ou d'une antenne à radiofréquence, ayant par exemple une conformation tubulaire, d'un dispositif capable de générer de la chaleur et/ou un rayonnement, par exemple dans le spectre infrarouge, ou des micro-ondes, etc., dans lequel ledit au moins un élément (30) a une conformation allongée et peut être logé à l'intérieur dudit piston (18), de ladite seconde aiguille (19) et peut parvenir à proximité de/ou être au moins partiellement logé dans ladite première aiguille (16) et/ou dans lequel ledit au moins un élément (30) comprend éventuellement un élément perforant placé dans une partie interne de celui-ci pour perforer ledit piston (18) et/ou dans lequel ledit au moins un élément (30) peut être logé dans ladite tige creuse (24) dudit piston (18) et être logé ou poussé dans un canal présent dans ladite tête (18a) dudit piston (18).

18. Kit (10, 100) selon l'une quelconque des revendications précédentes, dans lequel ledit premier élément de seringue (12) comprend une membrane perforable, située à ladite première extrémité (13a) dudit corps cylindrique (13), dans lequel ladite membrane perforable est adaptée pour être perforée par ladite seconde aiguille (19) dudit second élément de seringue (14) de manière à ce que ladite seconde aiguille vienne à proximité de ladite première aiguille (16).

19. Kit (10, 100) selon l'une quelconque des revendications précédentes, dans lequel ladite première aiguille (16, 116) fait face, lors de l'utilisation, vers l'extérieur dudit premier élément de seringue (12, 112) et/ou vers le site d'injection, tandis que ladite seconde aiguille (119) fait face, lors de l'utilisation, à ladite première cavité (113c) dudit corps cylindrique (113), dans lequel ladite première aiguille (16, 116) et ladite seconde aiguille (119) sont coaxiales.
